# EUROPEAN PATENT APPLICATION

(11) **EP 4 765 009 A1**
(43) Date of publication of application: **24.06.2026**
(21) Application number: 24859851.8
(22) Date of filing: 28.08.2024
(51) Int. Cl.: G06T 1/00, H04N 1/46

(54) **TAG AND METHOD FOR PROVIDING ARTICLE**

(30) Priority: 30.08.2023 WO PCT/JP2023/031559
(71) Applicant: Resonac Corporation, Tokyo 105-7325 (JP)
(72) Inventor: SAKAGUCHI, Suguru, Tokyo 105-7325 (JP); TOMITA, Miyuki, Tokyo 105-7325 (JP)
(74) Representative: Strehl & Partner mbB
(86) International application number: PCT/JP2024/030787
(87) International publication number: WO 2025/047821

(57) **Abstract**

A tag provided to an article has at least one of a first region indicating a color of the article perceived by a person with normal color vision in a mode perceivable by a person with color vision deficiency, and a second region indicating a color of the article perceived by a person with color vision deficiency in a mode perceivable by a person with normal color vision.

## Description

### Technical Field

An aspect of the present disclosure relates to a tag and a method for supplying an article.

### Background Art

Patent Literature 1 discloses an apparatus that outputs a color image. This apparatus extracts a region of a specific color that is difficult for a person with color vision deficiency to identify from a color image, synthesizes a character or a symbol clearly indicating the specific color in the extracted region, and outputs a synthesized color image.

### Citation List

### Patent Literature

[Patent Literature 1] Japanese Unexamined Patent Publication No. 2009-100312

### Summary of Invention

### Technical Problem

The present disclosure provides a technology for indicating a color of an article in consideration of color vision diversity.

### Solution to Problem

An aspect of the present disclosure is a tag provided to an article. The tag has at least one of a first region and a second region. The first region indicates a color of the article perceived by a person with normal color vision in a mode perceivable by a person with color vision deficiency. The second region indicates a color of the article perceived by a person with color vision deficiency in a mode perceivable by a person with normal color vision.

**In** this aspect, the color of the article perceived by the person with normal color vision is indicated in the mode perceivable by the person with color vision deficiency. Therefore, this tag can allow the person with color vision deficiency to recognize how the article appears to the person with normal color vision. Alternatively, in this aspect, the color of the article perceived by the person with color vision deficiency is indicated in the mode perceivable by the person with normal color vision. Therefore, this tag can allow the person with normal color vision to recognize how the article appears to the person with color vision deficiency. Thus, this tag can indicate the color of the article in consideration of color vision diversity.

Another aspect of the present disclosure is a method for supplying an article. The method for supplying an article includes a determining step, a providing step, and a supplying step. **In** the determining step, a tag is determined based on a color of the article. The tag has at least one of a first region indicating the color of the article perceived by a person with normal color vision in a mode perceivable by a person with color vision deficiency, and a second region indicating the color of the article perceived by a person with color vision deficiency in a mode perceivable by a person with normal color vision. **In** the providing step, the tag determined in the determining step is provided to the article. **In** the supplying step, the article to which the tag is provided is supplied.

The method according to this aspect can indicate the color of the article in consideration of color vision diversity by using the tag having the above characteristics.

### Advantageous Effects of Invention

According to various aspects of the present disclosure, it is possible to indicate a color of an article in consideration of color vision diversity.

### Brief Description of Drawings

FIG. 1 is a diagram showing an example of a garment equipped with a tag according to an embodiment.
Each of (A) to (E) of FIG. 2 is a diagram showing an example of the tag according to the embodiment.
FIG. 3 is a flowchart showing an example of a method for supplying an article.
FIG. 4 is a diagram showing an example of a configuration of a tag determination apparatus.
FIG. 5 is a diagram showing an example of a tag table.
FIG. 6 is a diagram showing an example of a configuration of a color conversion apparatus.
Each of (A) to (E) of FIG. 7 is a diagram showing an example of how to indicate a color in various embodiments.

### Description of Embodiments

Hereinafter, embodiments of the present disclosure will be described in detail with reference to the accompanying drawings. In the description of the drawings, the same or equivalent elements are denoted by the same reference numerals, and redundant description will be omitted.

### [Outline of Color Vision Diversity]

A concept according to the present disclosure is to provide a technology for indicating a color in consideration of color vision diversity. Color vision diversity refers to a situation in which recognition or discrimination of colors differs among people. It is difficult for people having different color visions to have a common recognition of colors. Therefore, it is important to deepen mutual understanding regarding recognition or discrimination of colors among people having different color visions.

As types of color vision, there are five types: C-type (Common), P-type (Protanope), D-type (Deuteranope), T-type (Tritanope), and A-type (Achromatic).

The C-type is a group having three types of cones: L cones, M cones, and S cones. The L cones are photoreceptor cells that respond with high sensitivity to light near long wavelengths such as red. The M cones are photoreceptor cells that respond with high sensitivity to light near medium wavelengths such as green. The S cones are photoreceptor cells that respond with high sensitivity to light near short wavelengths such as blue. In the case of Japanese people, 95% of males and 99% of females belong to the C-type. People of the C-type are also referred to as persons with normal color vision.

On the other hand, people of the P-type, D-type, T-type, and A-type are also referred to as persons with color vision deficiency. The P-type is a group composed of protanopia in which L cones are absent, and protanomaly in which sensitivity of L cones is shifted to resemble sensitivity of M cones. The D-type is a group composed of deuteranopia in which M cones are absent, and deuteranomaly in which sensitivity of M cones is shifted to resemble sensitivity of L cones. The T-type is a group having no S cones. The A-type is a group having only one type of cone or having no cones at all. Among persons with color vision deficiency, the P-type and D-type account for the majority, and proportions of the T-type and A-type are extremely small.

As an example of promoting the above-described common recognition or mutual understanding, the present disclosure provides a physical tag that allows a person with color vision deficiency to perceive a color recognized by a person with normal color vision, or allows a person with normal color vision to perceive a color recognized by a person with color vision deficiency. The tag may be designed such that common recognition or mutual understanding of colors is promoted among people of the C-type, P-type, and D-type. The present disclosure also provides an example in which a color perceived in the same manner by both a person with normal color vision and a person with color vision deficiency is used for an article.

### [Outline of Tag]

The tag is provided to an article and indicates information regarding a color of the article. The article is, for example, a garment, a cloth, furniture, an accessory, or the like, and is not particularly limited as long as it is an object having a color. The garment refers to anything worn on the body in general, and includes clothes, underwear, a hat, a scarf, gloves, footwear, and the like. Hereinafter, a case where the article is a garment will be described as an example. The tag being provided includes not only the tag being directly sewn onto the article but also the tag being attached to the article with a string like a price tag. Alternatively, the tag being provided may mean that the tag and the article are associated indirectly, that is, logically.

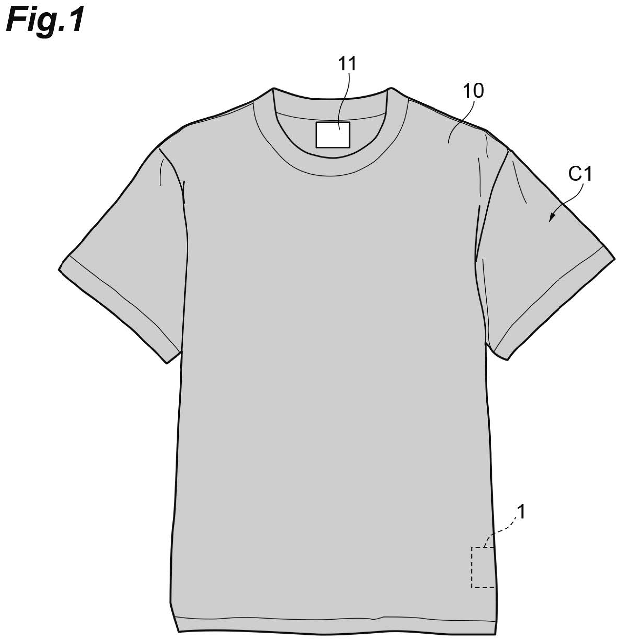

FIG. 1 is a diagram showing an example of a garment equipped with a tag according to an embodiment. As shown in FIG. 1, a tag 1 is provided to a garment 10. In the example shown in FIG. 1, the tag 1 is sewn onto a lining of the garment 10. Another tag 11 may be sewn onto the garment 10. The garment 10 has a color C1. The color C1 has a predetermined spectrum and is perceived as different colors by a person with normal color vision and a person with color vision deficiency. The spectrum is a plot of a relationship between wavelength (nm) and reflectance (%).

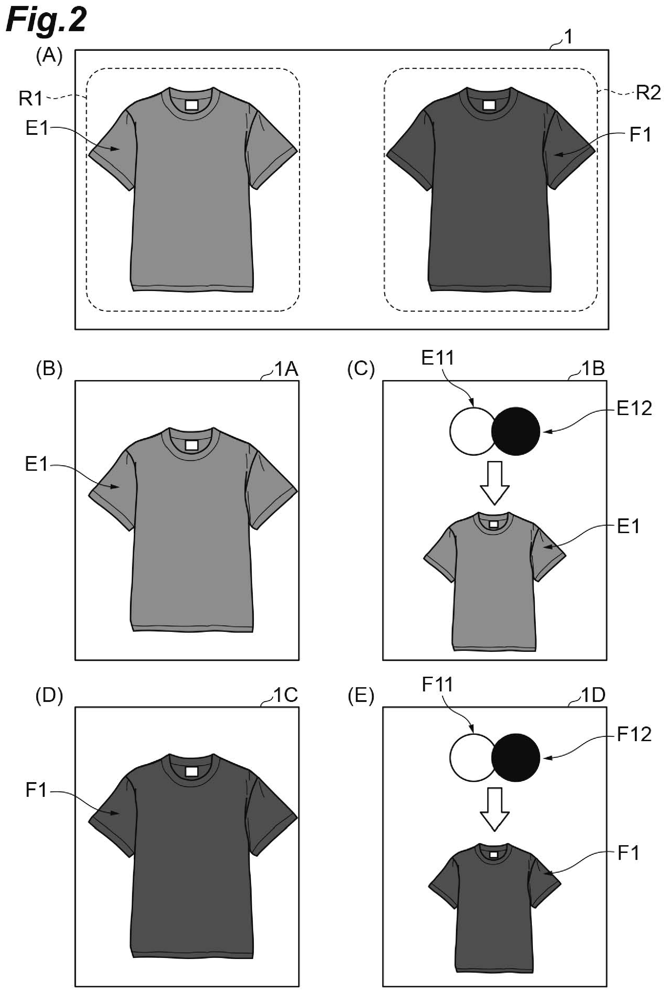

Each of (A) to (E) in FIG. 2 is a diagram showing an example of the tag according to the embodiment. The tag 1 shown in (A) of FIG. 2 has a first region R1 and a second region R2. The first region R1 is a region for displaying information for a person with color vision deficiency. The first region R1 includes information indicating how the color C1 appears to a person with normal color vision, for a person with color vision deficiency. As a more specific example, a figure of a garment having a first color E1 is displayed in the first region R1. The first color E1 has a spectrum configured such that a response of a color vision system of a person with color vision deficiency who has visually recognized the first region R1 matches a response of a color vision system of a person with normal color vision who has visually recognized the garment 10. "The responses of both match" means not only that a difference between the responses of both becomes 0, but also that the difference becomes equal to or less than a predetermined threshold. That is, the match includes a predetermined approximation range, and the threshold can be determined by a test. The first region R1 allows the person with color vision deficiency to recognize how the garment 10 appears to the person with normal color vision. The first region R1 may additionally include a character, a symbol, or the like indicating the first color E1. The first region R1 may be composed of a character, a symbol, or the like indicating the first color E1 without including the first color E1. For example, the first region R1 may include only characters such as "red" and "blue", or may include only symbols such as "R" and "G".

The second region R2 is a region for displaying information for a person with normal color vision. The second region R2 includes information indicating how the color C1 appears to a person with color vision deficiency, for a person with normal color vision. As a more specific example, a figure of a garment having a second color F1 is displayed in the second region R2. The second color F1 has a spectrum configured such that a response of a color vision system of a person with normal color vision who has visually recognized the second region R2 matches a response of a color vision system of a person with color vision deficiency who has visually recognized the garment 10. "The responses of both match" means not only that a difference between the responses of both becomes 0, but also that the difference becomes equal to or less than a predetermined threshold. That is, the match includes a predetermined approximation range, and the threshold can be determined by a test. The second region R2 allows the person with normal color vision to recognize how the garment 10 appears to the person with color vision deficiency. The second region R2 may additionally include a character, a symbol, or the like indicating the second color F1. The second region R2 may be composed of a character, a symbol, or the like indicating the second color F1 without including the second color F1. For example, the second region R2 may include only characters such as "red" and "blue", or may include only symbols such as "R" and "G".

The tag 1 only needs to have at least one of the first region R1 and the second region R2. For example, as shown in (B) of FIG. 2, since the tag 1A displays only information for a person with color vision deficiency, it can be said that the tag 1A has only the first region R1. The information displayed in the first region R1 may be displayed in various modes. For example, as shown in (C) of FIG. 2, the tag 1B includes a plurality of colors E11 and E12 perceivable by a person with color vision deficiency. The plurality of colors E11 and E12 are colors that become the first color E1 perceived by a person with normal color vision by being mixed. For example, when the first color E1 is pink, the color E11 can be white and the color E12 can be red. Thus, colors displayed as sources of combination can be simplified (made into primary colors). Therefore, the tag 1B can indicate the first color E1 perceived by a person with normal color vision in a mode easy to recognize for a person with color vision deficiency.

Each of the plurality of colors E11 and E12 may be a color in which a difference between a response of a color vision system of a person with color vision deficiency and a response of a color vision system of a person with normal color vision falls within a predetermined range. That is, each of the plurality of colors E11 and E12 is a color that can be perceived as substantially the same color by both a person with color vision deficiency and a person with normal color vision. The predetermined range can be determined by a test. Hereinafter, in the present disclosure, a color that can be perceived as substantially the same color by both a person with color vision deficiency and a person with normal color vision is referred to as a universal color. By using the universal color, the tag 1B can allow even a person with normal color vision to understand the information for a person with color vision deficiency described on the tag 1B.

The tag 1 may have only the second region R2. As shown in (D) of FIG. 2, since the tag 1C displays only information for a person with normal color vision, it can be said that the tag 1C has only the second region R2. The information displayed in the second region R2 may be displayed in various modes. For example, as shown in (E) of FIG. 2, the tag 1D includes a plurality of colors F11 and F12 perceivable by a person with normal color vision. The plurality of colors F 11 and F12 are colors that become the second color F1 perceived by a person with color vision deficiency by being mixed. For example, when the second color F1 is sky blue, the color F11 can be white and the color F12 can be blue. Thus, colors displayed as sources of combination are simplified (made into primary colors). Therefore, the tag 1D can indicate the second color F1 perceived by a person with color vision deficiency in a mode easy to recognize for a person with normal color vision.

Each of the plurality of colors F11 and F12 may be a color in which a difference between a response of a color vision system of a person with color vision deficiency and a response of a color vision system of a person with normal color vision falls within a predetermined range. The predetermined range can be determined by a test. By using the universal color, the tag 1D can allow even a person with color vision deficiency to understand the information for a person with normal color vision described on the tag 1D.

The plurality of colors E11 and E12 described using (C) of FIG. 2 and the plurality of colors F11 and F12 described using (E) of FIG. 2 are also applicable to the tag 1 shown in (A) of FIG. 2. Therefore, a total of six types of tags are exemplified: the tag 1 having the first region R1 and the second region R2; a tag (not shown) providing a display of a combination of a plurality of colors in the tag 1; the tag 1A having only the first region R1; the tag 1B providing a display of a combination of a plurality of colors in the tag 1A; the tag 1C having only the second region R2; and the tag 1D providing a display of a combination of a plurality of colors in the tag 1C.

Note that in order to acquire information regarding a color from a tag, a person who intends to perceive the color of the garment 10 needs to know whether the tag includes the first region R1 or the second region R2, or includes both regions. When the tag includes both regions, the person who intends to perceive the color of the garment 10 needs to know which region is the first region R1 and which region is the second region R2. These pieces of information may be described on the tag, may be separately provided to the article as explanatory information, or may be made known by being posted at a sales floor of the article or the like.

### [Outline of Method for Supplying Article]

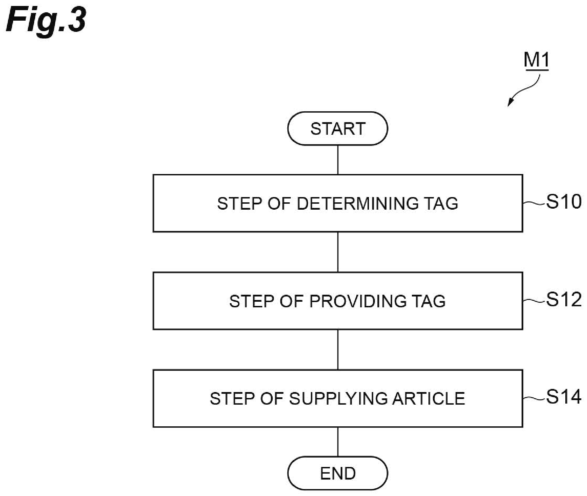

FIG. 3 is a flowchart showing an example of a method for supplying an article. The supplying method M1 shown in FIG. 3 includes a step of determining a tag (step S10), a step of providing the tag (step S12), and a step of supplying the article (step S14).

**In** the step of determining a tag (step S10), an apparatus or a person determines a tag to be provided to an article based on a color of the article. First, a case where an apparatus determines a tag will be described.

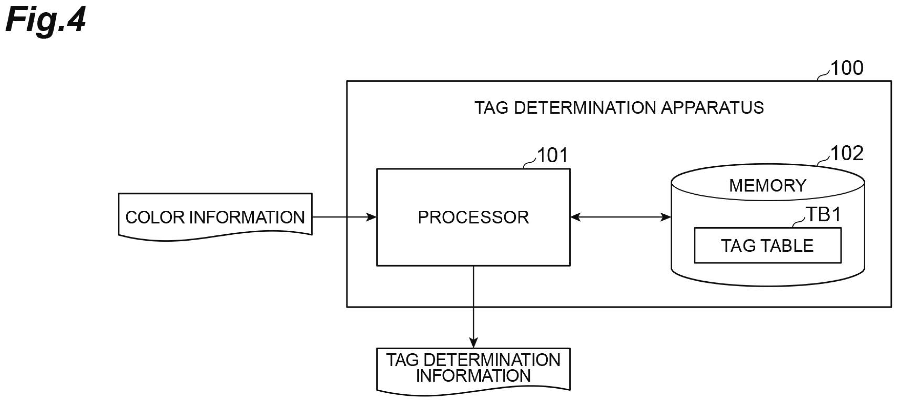

FIG. 4 is a diagram showing an example of a configuration of a tag determination apparatus. The tag determination apparatus 100 shown in FIG. 4 is configured by one computer or a plurality of computers connected to each other via a communication network. The computer generally includes a processor, a memory, a communication interface, an input device, and an output device as hardware devices. Examples of the processor include a CPU and a GPU. The memory is configured by a flash memory, a hard disk, or the like. The communication interface is configured by a network card or a wireless communication module. Examples of the input device include a keyboard, a pointing device, a touch panel, a microphone, a sensor, and a camera. Examples of the output device include a monitor, a touch panel, a head-mounted display (HMD), and a speaker. The tag determination apparatus 100 includes a processor 101 and a memory 102 as an example. The memory 102 stores a tag table TB1 in advance.

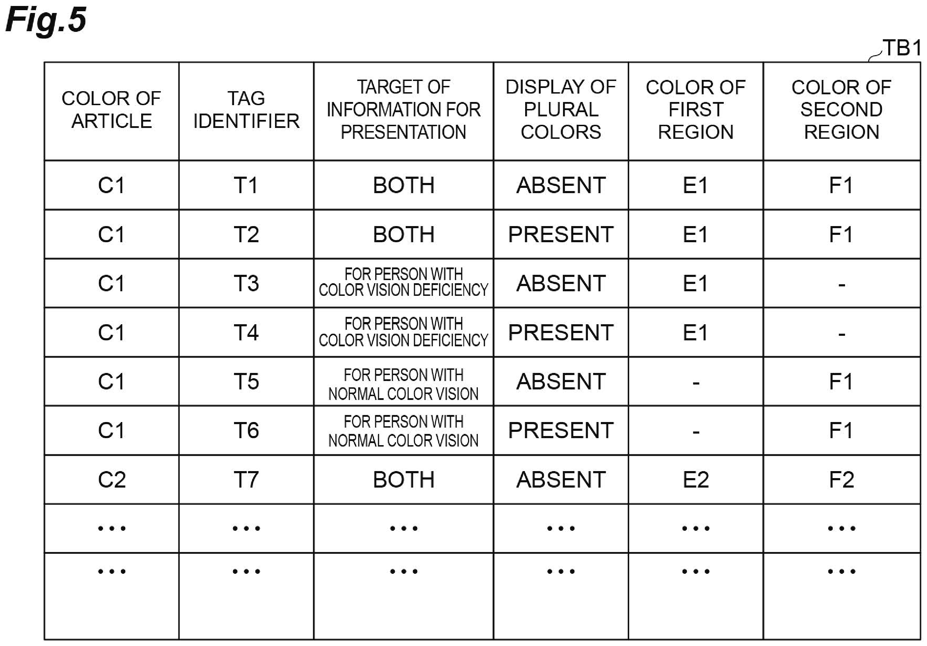

FIG. 5 is a diagram showing an example of a tag table. The tag table TB1 shown in FIG. 5 is an example of a table in a case where the above-described six types of tags are prepared for each color. As shown in FIG. 5, the tag table TB1 is a table in which "Color of Article" and "Tag Identifier" are associated. "Tag Identifier" is further associated with "Target of Information Presentation", "Display of Plural Colors", "Color of First Region", and "Color of Second Region".

In the example shown in FIG. 5, "Color of Article" indicates the color of the garment 10. "Tag Identifier" is information that uniquely identifies a tag. "Target of Information Presentation" is information indicating to whom the information displayed on the tag is directed. Here, it is classified into three: information for a person with color vision deficiency, information for a person with normal color vision, and information for both. "Display of Plural Colors" is information indicating presence or absence of a mode of displaying with a combination of a plurality of colors. "Color of First Region" is a color displayed in the first region R1. "Color of Second Region" is a color displayed in the second region R2.

Subsequently, an operation of the tag determination apparatus 100 will be described. First, the processor 101 accepts color information of the garment 10. The processor 101 may accept the color information by being input by a user of the tag determination apparatus 100, or may accept color information recognized by an image sensor imaging the garment 10. Subsequently, the processor 101 refers to the tag table TB1 in the memory 102 and determines a tag based on the accepted color information. The processor 101 outputs the determined tag as tag determination information.

For example, when the processor 101 accepts the color of the article "C1", the processor 101 refers to the tag table TB1 and acquires tag identifiers "T1" to "T6" associated with the color of the article "C1". The processor 101 selects one tag identifier from the tag identifiers "T1" to "T6" and outputs it as the tag determination information. For example, the processor 101 inquires of the user of the tag determination apparatus 100 about "Target of Information Presentation" and "Display of Plural Colors", and selects one tag identifier based on a response of the user. For example, when the target of information presentation is "for person with color vision deficiency" and the display of plural colors is "absent" in the response of the user, the processor 101 selects the tag identifier "T3". Thereby, the tag 1A shown in (B) of FIG. 2 is selected.

The processor 101 may select one tag identifier using "Target of Information Presentation" and "Display of Plural Colors" input in advance by the user. The processor 101 may randomly select one tag identifier. With the above operation, the step of determining a tag (step S10) shown in FIG. 3 ends. When a person executes the step of determining a tag (step S10), the person may select a tag corresponding to the color of the article with reference to, for example, the tag table TB1 shown in FIG. 5.

When the step of determining a tag ends, the step of providing the tag (step S12) is executed. In the step of providing the tag, an apparatus or a person provides the tag determined in step S10 to the garment 10. When the step of providing the tag ends, the step of supplying the article (step S14) is executed. In the step of supplying the article, an apparatus or a person supplies the garment 10 to which the tag is provided. Supplying includes transportation, display, transfer, sale, and the like.

The flowchart shown in FIG. 3 ends here. By executing the flowchart shown in FIG. 3, a tag corresponding to the color of the article is provided to the article.

### [Color Displayed on Tag]

The colors displayed in the first region R1 and the second region R2 are determined based on the color of the article. Hereinafter, a configuration and an operation of an apparatus that determines the colors displayed in the first region R1 and the second region R2 will be outlined. Hereinafter, the color of the article is referred to as a base color, the color of the first region R1 is referred to as a first color, and the color of the second region R2 is referred to as a second color. The first color is a color having a spectrum configured such that a response of a color vision system of a person with color vision deficiency who has visually recognized the first region R1 matches a response of a color vision system of a person with normal color vision who has perceived the base color. The second color is a color having a spectrum configured such that a response of a color vision system of a person with normal color vision who has visually recognized the second region R2 matches a response of a color vision system of a person with color vision deficiency who has perceived the base color.

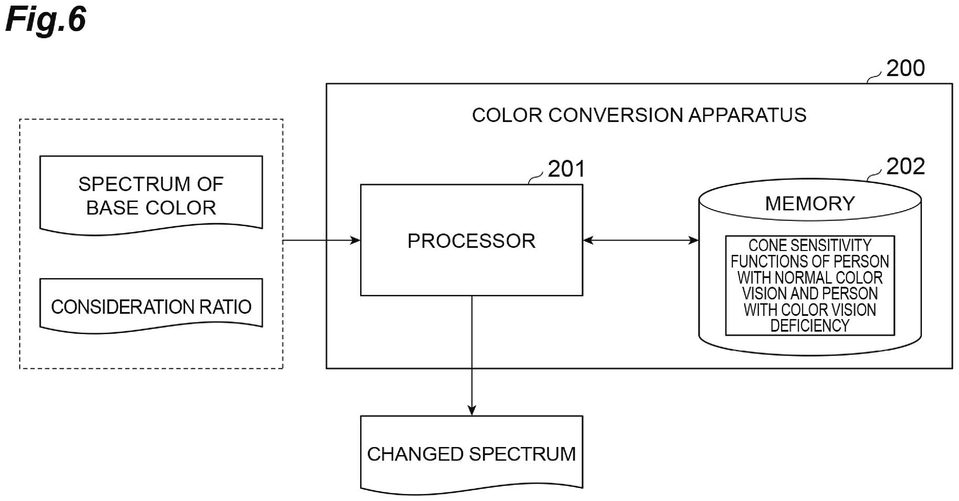

FIG. 6 is a diagram showing an example of a configuration of a color conversion apparatus. The color conversion apparatus 200 shown in FIG. 6 is configured by one computer or a plurality of computers connected to each other via a communication network. The color conversion apparatus 200 includes a processor 201 and a memory 202. The memory 202 stores cone sensitivity functions of a person with normal color vision and cone sensitivity functions of a person with color vision deficiency in advance. The cone sensitivity functions are also referred to as a color vision system, are sensitivity characteristics of respective L cones, M cones, and S cones, and indicate a relationship between wavelength and relative sensitivity. The cone sensitivity function outputs a response according to a shape of an input spectrum. The cone sensitivity functions may be prepared for each type of color vision; for example, P-type cone sensitivity functions and D-type cone sensitivity functions may be prepared.

The processor 201 inputs a spectrum of the base color in the visible light region. Then, the processor 201 acquires respective responses (three numbers) of L cones, M cones, and S cones based on the spectrum of the base color in the visible light region and the cone sensitivity functions of the person with normal color vision stored in the memory 202. Subsequently, the processor 201 acquires respective responses (three numbers) of L cones, M cones, and S cones based on the spectrum of the base color in the visible light region and the cone sensitivity functions of the person with color vision deficiency stored in the memory 202. Then, the processor 201 repeats changing the shape of the spectrum of the base color and acquiring the corresponding response of the person with color vision deficiency so that the response of the person with color vision deficiency approaches (matches) the response of the person with normal color vision. Thereby, a spectrum in which the response of the person with color vision deficiency and the response of the person with normal color vision match is obtained. The color of the spectrum obtained in this manner is the above-described first color.

The spectrum of the second color can be determined in the same manner as the first color. That is, the processor 201 repeats changing the shape of the spectrum of the base color and acquiring the corresponding response of the person with normal color vision so that the response of the person with normal color vision approaches (matches) the response of the person with color vision deficiency. Thereby, a spectrum in which the response of the person with normal color vision and the response of the person with color vision deficiency match is obtained. The color of the spectrum obtained in this manner is the above-described second color.

Next, the universal color which is a plurality of colors displayed in the first region R1 and the second region R2 will be described. The universal color can be generated based on two colors and a consideration ratio. The consideration ratio is a degree of consideration of color vision diversity, and is a numerical value within a range of 0% to 100%. The two colors are a color with a consideration ratio of 0% and a color with a consideration ratio of 100%. The color with a consideration ratio of 0% is the base color. The color with a consideration ratio of 100% is a color in which the response of the color vision system of the person with color vision deficiency matches the response of the color vision system of the person with normal color vision who has perceived the base color, and is the above-described first color. The processor 201 inputs the consideration ratio, linearly combines the spectrum of the base color (or a color obtained by changing the spectrum of the base color) and the spectrum of the first color according to the consideration ratio so as to achieve the input consideration ratio, and outputs a spectrum changed to correspond to the consideration ratio. The universal color can be generated with a consideration ratio of 20% or a value close thereto as an example. The processor 201 may output a spectrum changed to correspond to the consideration ratio in a different manner. For example, the processor 201 prepares a color in which the shape of the spectrum is changed so that the response of the person with color vision deficiency approaches (matches) the response of the person with normal color vision under a condition that RGB of the base color is kept constant. When creating a color having a larger consideration ratio than the prepared color, the processor 201 linearly combines the prepared color and the color with a consideration ratio of 100% according to the consideration ratio, and outputs a spectrum changed to correspond to the consideration ratio.

### [Various Embodiments]

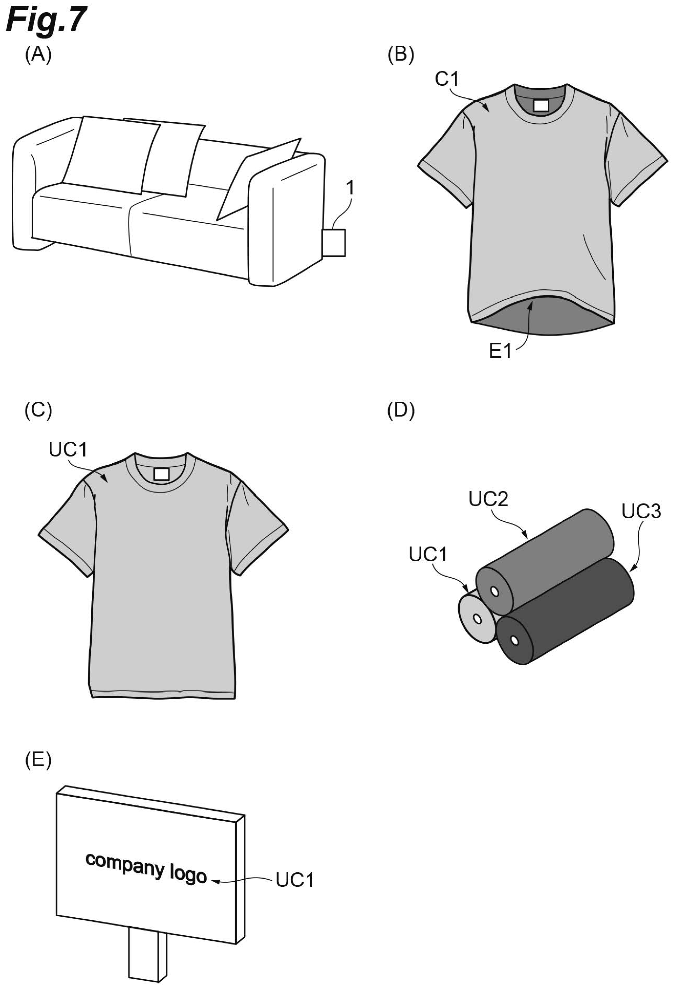

Various embodiments of the technology for indicating a color in consideration of color vision diversity will be described. Each of (A) to (E) of FIG. 7 is a diagram showing an example of how to indicate a color in various embodiments. The tag 1 shown in (A) of FIG. 2 and the like is not limited to the garment 10, and can also be provided to furniture such as a sofa as shown in (A) of FIG. 7. Alternatively, the tag may be provided to an accessory such as stationery. Thereby, mutual understanding between a person with color vision deficiency and a person with normal color vision can be deepened regarding the color of the furniture or the accessory as well.

The first color shown in the present disclosure may be applied to a garment without using a tag. For example, as shown in (B) of FIG. 7, a lining of a garment of color C1 can be made the first color E1. Thereby, in a reversible garment, a relationship can be given to colors of front and back, and mutual understanding between a person with color vision deficiency and a person with normal color vision can be deepened.

The universal color shown in the present disclosure can be applied to various articles. For example, as shown in (C) of FIG. 7, a universal color UC1 may be adopted for a color of a garment. Alternatively, as shown in (D) of FIG. 7, universal colors UC1, UC2, and UC3 may be adopted for colors of a cloth. Furthermore, as shown in (E) of FIG. 7, a corporate logo or the like may be described in the universal color UC1. Thereby, a difference in color recognition between a person with color vision deficiency and a person with normal color vision can be reduced.

### [Modification Examples]

The embodiments of the present disclosure have been described in detail above. However, the present disclosure is not limited to the above embodiments. The present disclosure can be modified in various ways without departing from the gist thereof.

**In** the tags shown in (C) of FIG. 2 and (E) of FIG. 2, the first color E1 and the second color F1 are displayed together with the plurality of colors, but only the plurality of colors may be displayed.

**In** FIG. 5, an example in which six types of tags are prepared for one color has been described, but the number of types of tags for one color is not particularly limited, and for example, there may be only one type of tag for one color. When there is only one type of tag for one color, the tag table TB1 shown in FIG. 5 may be composed of only columns of "Color of Article" and "Tag Identifier".

The first region R1 and the second region R2 may indicate a color indirectly using a QR code (registered trademark) or the like.

When the universal color is not adopted, the color conversion apparatus 200 does not need to acquire and use the consideration ratio. When the P-type cone sensitivity functions and the D-type cone sensitivity functions are stored in the memory 202, the color conversion apparatus 200 may accept information on the person with color vision deficiency and determine cone sensitivity functions to be used.

Articles according to the present disclosure are classified into various categories. The categories include, for example, food, clothing, and housing, clothing/fashion, school/children, brand/advertising, toys, and others. Examples of specific articles included in these categories are as follows. Articles related to food, clothing, and housing are, for example, clothes, foods containing colorants, tableware, tablecloths, wallpaper, furniture, bedding, and the like. Articles related to food, clothing, and housing may be a combination of wallpaper, furniture, bedding, and the like with lighting. Articles related to clothing/fashion are, for example, umbrellas, hats, hairpins, hair ties, headbands, hair bands, glasses, sunglasses, clip-on earrings, pierced earrings, earphones, cosmetics, articles related to nail art (nail polish, gel, stickers, and the like), necklaces, neckties, scarves, winter scarves, shawls, tie pins, innerwear tops, outerwear tops, innerwear bottoms, outerwear bottoms, gloves, wristbands, bracelets, watches, smartwatches, rings, smartphone cases, bags, belts, socks, leggings, tights, stockings, anklets, shoes, sandals, geta, slippers, round fans, folding fans, handkerchiefs, towels, and the like. Articles related to school/children are, for example, stationery, notebooks, letter sets, toolboxes, school bags, textbooks/teaching materials, uniforms, gym clothes, indoor shoes, paint sets, lunch boxes, crayons, sticky notes, files, binders, and the like. Articles related to brand/advertising are, for example, articles with logos, signboards, articles related to advertising, packages (beverages, confectionery, book covers, CD jackets), and the like. Articles related to toys are, for example, stuffed animals/dolls, playing house items, clay, and the like. Other articles are, for example, candles, soaps, business cards, and the like. Thus, articles according to the present disclosure are diverse, and specific articles are included in each category. This makes it possible to accommodate various uses or scenes.

### [Appendix]

As will be appreciated from the various examples above, the present disclosure includes the following aspects.

### (Appendix 1)

A tag provided to an article, comprising:
at least one of a first region indicating a color of the article perceived by a person with normal color vision in a mode perceivable by a person with color vision deficiency, and a second region indicating a color of the article perceived by a person with color vision deficiency in a mode perceivable by a person with normal color vision.

### (Appendix 2)

The tag according to Appendix 1, wherein the first region includes a color having a spectrum configured such that a response of a color vision system of a person with color vision deficiency who has visually recognized the first region matches a response of a color vision system of a person with normal color vision who has visually recognized the article.

### (Appendix 3)

The tag according to Appendix 1 or 2, wherein the second region includes a color having a spectrum configured such that a response of a color vision system of a person with normal color vision who has visually recognized the second region matches a response of a color vision system of a person with color vision deficiency who has visually recognized the article.

### (Appendix 4)

The tag according to any one of Appendices 1 to 3, wherein the first region includes a plurality of colors perceivable by a person with color vision deficiency, the plurality of colors becoming the color of the article perceived by a person with normal color vision by mixing the plurality of colors.

### (Appendix 5)

The tag according to Appendix 4, wherein each of the plurality of colors perceivable by a person with color vision deficiency is a color in which a difference between a response of a color vision system of a person with color vision deficiency and a response of a color vision system of a person with normal color vision falls within a predetermined range.

### (Appendix 6)

The tag according to any one of Appendices 1 to 5, wherein the second region includes a plurality of colors perceivable by a person with normal color vision, the plurality of colors becoming the color of the article perceived by a person with color vision deficiency by mixing the plurality of colors.

### (Appendix 7)

The tag according to Appendix 6, wherein each of the plurality of colors perceivable by a person with normal color vision is a color in which a difference between a response of a color vision system of a person with color vision deficiency and a response of a color vision system of a person with normal color vision falls within a predetermined range.

### (Appendix 8)

The tag according to Appendix 1 or 2, wherein the tag has the first region.

### (Appendix 9)

The tag according to any one of Appendices 1 to 8, wherein the article is a garment.

### (Appendix 10)

A method for supplying an article, comprising:
a step of determining a tag based on a color of the article, wherein the tag has at least one of a first region indicating the color of the article perceived by a person with normal color vision in a mode perceivable by a person with color vision deficiency, and a second region indicating the color of the article perceived by a person with color vision deficiency in a mode perceivable by a person with normal color vision;
a step of providing the tag determined in the determining step to the article; and
a step of supplying the article to which the tag is provided.

### (Appendix 11)

The method for supplying an article according to Appendix 10, wherein the tag has the first region.

### (Appendix 12)

The method for supplying an article according to Appendix 10 or 11, wherein the article is a garment.

According to the tag according to Appendix 1, the color of the article perceived by the person with normal color vision is indicated in the mode perceivable by the person with color vision deficiency. Therefore, this tag can allow the person with color vision deficiency to recognize how the article appears to the person with normal color vision. The person with color vision deficiency will not make a mistake in selecting the color of the article, and it is possible to give the person with color vision deficiency confidence in selecting the color. Alternatively, according to this tag, the color of the article perceived by the person with color vision deficiency is indicated in the mode perceivable by the person with normal color vision. Therefore, this tag can allow the person with normal color vision to recognize how the article appears to the person with color vision deficiency. Thus, this tag can indicate the color of the article in consideration of color vision diversity.

According to Appendix 2, the tag can allow the person with color vision deficiency to perceive how the article appears to the person with normal color vision by color.

According to Appendix 3, the tag can allow the person with normal color vision to perceive how the article appears to the person with color vision deficiency by color.

According to Appendix 4, the tag can indicate the color of the article perceived by the person with normal color vision in a mode easy to recognize for the person with color vision deficiency.

According to Appendix 5, by adopting a color with a small difference in recognition between the person with color vision deficiency and the person with normal color vision, the tag can allow even the person with normal color vision to understand the information for the person with color vision deficiency described on the tag.

According to Appendix 6, the tag can indicate the color of the article perceived by the person with color vision deficiency in a mode easy to recognize for the person with normal color vision.

According to Appendix 7, by adopting a color with a small difference in recognition between the person with color vision deficiency and the person with normal color vision, the tag can allow even the person with color vision deficiency to understand the information for the person with normal color vision described on the tag.

According to Appendix 8, the tag can allow the person with color vision deficiency to recognize how the article appears to the person with normal color vision.

According to Appendix 9, the tag can allow the person with color vision deficiency to recognize how the garment appears to the person with normal color vision. Alternatively, the tag can allow the person with normal color vision to recognize how the garment appears to the person with color vision deficiency.

The method according to Appendix 10 can indicate the color of the article in consideration of color vision diversity by using a tag having the above characteristics.

The method according to Appendix 11 can allow a person with color vision deficiency to recognize how the article appears to a person with normal color vision.

The method according to Appendix 12 can allow a person with color vision deficiency to recognize how the garment appears to a person with normal color vision. Alternatively, the method according to Appendix 12 can allow a person with normal color vision to recognize how the garment appears to a person with color vision deficiency.

### Reference Signs List

1, 1A, 1B, 1C, 1D: Tag; R1: First region; R2: Second region.

## Claims

1. A tag provided to an article, comprising:
at least one of a first region indicating a color of the article perceived by a person with normal color vision in a mode perceivable by a person with color vision deficiency, and a second region indicating a color of the article perceived by a person with color vision deficiency in a mode perceivable by a person with normal color vision.

2. The tag according to claim 1, wherein the first region includes a color having a spectrum configured such that a response of a color vision system of a person with color vision deficiency who has visually recognized the first region matches a response of a color vision system of a person with normal color vision who has visually recognized the article.

3. The tag according to claim 1 or 2, wherein the second region includes a color having a spectrum configured such that a response of a color vision system of a person with normal color vision who has visually recognized the second region matches a response of a color vision system of a person with color vision deficiency who has visually recognized the article.

4. The tag according to claim 1 or 2, wherein the first region includes a plurality of colors perceivable by a person with color vision deficiency, the plurality of colors becoming the color of the article perceived by a person with normal color vision by mixing the plurality of colors.

5. The tag according to claim 4, wherein each of the plurality of colors perceivable by a person with color vision deficiency is a color in which a difference between a response of a color vision system of a person with color vision deficiency and a response of a color vision system of a person with normal color vision falls within a predetermined range.

6. The tag according to claim 1, wherein the second region includes a plurality of colors perceivable by a person with normal color vision, the plurality of colors becoming the color of the article perceived by a person with color vision deficiency by mixing the plurality of colors.

7. The tag according to claim 6, wherein each of the plurality of colors perceivable by a person with normal color vision is a color in which a difference between a response of a color vision system of a person with color vision deficiency and a response of a color vision system of a person with normal color vision falls within a predetermined range.

8. The tag according to claim 1 or 2, wherein the tag has the first region.

9. The tag according to claim 1 or 2, wherein the article is a garment.

10. A method for supplying an article, comprising:
a step of determining a tag based on a color of the article, wherein the tag has at least one of a first region indicating the color of the article perceived by a person with normal color vision in a mode perceivable by a person with color vision deficiency, and a second region indicating the color of the article perceived by a person with color vision deficiency in a mode perceivable by a person with normal color vision;
a step of providing the tag determined in the determining step to the article; and
a step of supplying the article to which the tag is provided.

11. The method for supplying an article according to claim 10, wherein the tag has the first region.

12. The method for supplying an article according to claim 10 or 11, wherein the article is a garment.
